# EUROPÄISCHE PATENTSCHRIFT

(11) **EP 2 209 772 B1**
(45) Veröffentlichungstag und Bekanntmachung des Hinweises auf die Patenterteilung: **02.09.2015**
(21) Anmeldenummer: 08850140.8
(22) Anmeldetag: 14.11.2008
(51) Int. Cl.: C08G 63/85, C07D 319/12

(54) **VERFAHREN ZUR HERSTELLUNG ZYKLISCHER DIESTER VON L-, D- UND D,L-MILCHSÄURE**
METHOD FOR PRODUCING CYCLIC DIESTERS OF L-, D- AND D, L-LACTIC ACID
PROCÉDÉ DE PRODUCTION DE DIESTERS CYCLIQUES DE L'ACIDE L-, D- ET D,L-LACTIQUE

(30) Priorität: 14.11.2007 AT 18382007
(43) Veröffentlichungstag der Anmeldung: 28.07.2010
(73) Patentinhaber: JUNGBUNZLAUER AUSTRIA AG, 1010 Wien (AT)
(72) Erfinder: RAFLER, Gerald, 14473 Potsdam (DE); RAFLER, Jutta, 14473 Potsdam (DE); WINDSPERGER, Andreas, A-3034 Maria Anzbach (AT); EDLAUER, Robert, A-2136 Laa a.d. Thaya (AT); GASS, Josef, A-2265 Drösing (AT)
(74) Vertreter: Ellmeyer, Wolfgang
(86) Internationale Anmeldenummer: PCT/AT2008/000413
(87) Internationale Veröffentlichungsnummer: WO 2009/062224

(56) Entgegenhaltungen:
- EP-A- 0 261 572
- WO-A-01/81610

## Beschreibung

Die Erfindung betrifft ein Verfahren zur effizienten Herstellung von Dilactiden hoher chemischer und/oder optischer Reinheit durch selektive Katalyse der Polykondensation von Milchsäure zu Polymilchsäure mit anschließender zyklisierender Depolymerisation des Polyesters zum zyklischen Dimer.

### Stand der Technik

Die internationale Entwicklung am Kunststoffmarkt zeigt, dass Poly-L-milchsäure (PLA) mit ihrem thermoplasttypischen Eigenschaftsbild, den für Thermoplaste und insbesondere Heterokettenpolymere typischen Möglichkeiten zur einsatzspezifischen Adaption der Materialeigenschaften durch Compoundierung und chemische Modifizierung, der biogenen Rohstoffbasis in Kombination mit der biologischen Abbaubarkeit des Polymers die besten Chancen auf dem Markt für alternative Kunststoffe besitzt. Es ist zu erkennen, dass diesem Polymerwerkstoff nicht nur auf Einsatzfeldern mit temporär geforderter Stabilität und schwieriger Rezyklierbarkeit, wie bei
- Hygienematerialien (Babywindeln, Inkontinenzartikeln),
- ausgewählten Verpackungsmitteln, insbesondere Schalen für Obst, Fleisch- und Wurstwaren sowie Fisch,
- diversen Medizinprodukten mit Einwegcharakter und
- Folien für die Land- und Gartenwirtschaft sowie Pflanzcontainern,
ein breiter Markt mit hohem Wachstumspotenzial offen steht, sondern auch bei Synthesefaserstoffen für hochwertige Funktionsbekleidung (Sport, Freizeit) sowie als Polymergranulat für die Spritzguss- und Extrusionsverformung zu langzeitstabilen Formkörpern werden PLA realistische Chancen zur Substitution konventioneller Polyestermaterialien eingeräumt. Daneben sind Biomaterialien auf Basis PLA für chirurgische und galenische Anwendungen ein attraktiver Zukunftsmarkt im Sektor sehr hochpreisiger High-Tech-Medizinprodukte.

Dieses erweiterte Applikationsprofil von PLA-Kunststoffen erfordert hochreine Ausgangsstoffe für die Herstellung dieses Polymers sowie vielfältige molekulare Adaptionen der molekularen Parameter des Polymers an die applikativen Anforderungen. Dies betrifft beispielsweise die Molmasse und ihre Verteilung, Vermeidung der Bildung von Fehlstrukturen, Erhalt bzw. Steuerung der Stereoisomerie etc.

Die Herstellung des Polymers erfolgt in einem mehrstufigen Prozess mit biotechnologischen und chemischen Verfahrenstufen, der im Wesentlichen umfasst:
- Hydrolyse stärkehaltiger Substrate zu Glucose;
- Fermentation von Glucose zu L-Milchsäure;
- Polykondensation zu einer niedermolekularen Poly-L-milchsäure;
- zyklisierende Depolymerisation zu L,L-Dilactid;
- Ringöffnungspolymerisation des L,L-Dilactids zu Poly-L-milchsäure;
- Stabilisierung und Entmonomerisierung des Polymers.

Die direkte Polykondensation der L-Milchsäure gelingt für hochmolekulare Polyester nur unter Laborbedingungen mittels einer Azeotropmethode in einem Lösungsmittel für das Monomer und das Polymer. Technische Verfahren müssen infolge der Lage des Ring/Ketten-Gleichgewichts von linearen und zyklischen Oligomeren den beschriebenen mehrstufigen Prozess mit niedermolekularer Poly-L-milchsäure und L,L-Dilactid als Zwischenstufen anwenden (vgl. Schema 1).

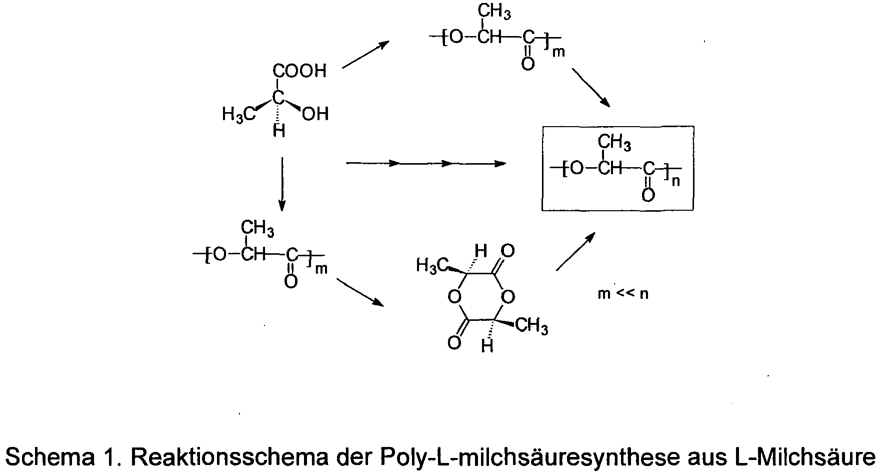

Dieser mehrstufige Prozess der Herstellung von Poly-L-milchsäure wird in einer Vielzahl von Patenten vor allem der Cargill Inc. USA (vgl. beispielsweise US 6.277.951, US 6.005.067, US 5.357.035, US 6.291.597, CA 2.128.509), der Dainippon Ink & Chem. Japan (vgl. beispielsweise US 5.844.066, US 5.616.657, US 5.605.981, US 5.403.897), Mitsui Toatsu Japan (vgl. beispielsweise US 5.194.473), Neste Oy Finnland (WO 98/36008), Brussels Biotec (vgl. z.B. GB 2.407.572, WO 98/02480, DE 69905016, US 6.489.508, US 2004/0014991) oder der Shimadzu Japan (vgl. beispielsweise US 5.770.682, US 5.866.677, JP 7206851) beschrieben.

Unabhängig von technologischen und apparatetechnischen Konzepten in den aufgeführten Patentschriften ist die Gewinnung eines polymerisationsfähigen L,L-Dilactids zentraler Bestandteil bei den beschriebenen Verfahren. Chemische und chirale bzw. stereochemische Reinheit (bei L,L-, D,D- bzw. meso-Dilactid) des aus der fermentativ (L- bzw. D-Milchsäure) bzw. chemisch erzeugten Milchsäure (D,L-Milchsäure) gebildeten Dilactids sind essenziell für die Herstellung einer hochmolekularen Polymilchsäure mit entsprechenden morphologischen und thermischen Eigenschaften. D-Lactideinheiten in der Poly-L-milchsäure, eingebracht durch unvollständige Abtrennung von D,D-Dilactid oder meso-Dilactid bei der Aufarbeitung des L,L-Dilactids, wirken trotz ihrer chemisch gleichen Struktur wie Comonomere und beeinflussen die Taktizität des Makromoleküls. Dies führt dann zu Änderungen in der Morphologie des polymeren Festkörpers mit den beobachteten makroskopischen Änderungen im Schmelz- und Erweichungsverhalten sowie ausgewählten mechanischen Eigenschaften. Dabei sind die morphologischen Änderungen direkt eine Funktion des Anteils an D-Lactideinheiten im Polymer (zu morphologischen und mechanischen Eigenschaften von PLA vgl. beispielsweise D.W. Grijpma et al., Makromol. Chemie 195, 1649 (1994), G. Perego et al., J. Appl. Polymer Sci. 59, 37 (1996), G. Schmack et al., J. Appl. Polymer Sci. 73, 2785 (1999), R. E. Drumright et al., Adv. Mater 12 H 23, 1841 (2000), R. Auras et al., Macromol. Biosci. 4, 835 (2004)).

Die Stereoisomere des L,L-Dilactids sind als "Verunreinigungen" aufzufassen, und sie müssen aufwendig abgetrennt werden. Die Verteilung der stereoisomeren L,L-, D,D- und meso-Dilactide bei der Depolymerisation eines Poly-L-lactids mit D-Monomeranteilen in der Kette lässt sich für vollständigen Umsatz und mit p für den L-Lactidgehalt in der Kette nach den Gleichungen (1 a) bis (1 c) berechnen:

L,L-Dilactid: p² (1a)

meso-Dilactid: 2p(1-p) (1b)

D,D-Dilactid: (1-p)² (1c)

Während die Bildung von D,D-Dilactid bei D-Anteilen unter 10 % (entsprechend p > 0,9 für L-Monomeranteil) zu vernachlässigen ist, kann meso-Dilactid in diesem Bereich in Mengen gebildet werden, die die Ausbeute erheblich reduzieren und die Aufarbeitung des L-Monomers erschweren.

Technologie der Herstellung sowie die Reinigung des Monomers einschließlich der eingesetzten Apparatetechnik dominieren deshalb die beanspruchten Schutzrechte innerhalb des Gesamtprozesses. Effektivität und Selektivität der zyklisierenden Depolymerisation der Oligo-L-milchsäure sowie Reinigungsverfahren für das Rohlactid betreffen deshalb in gleicher Weise die Schutzansprüche in den aufgeführten Patentschriften. Beschrieben werden zumeist destillative Reinigungsverfahren unter Vakuum (vgl. beispielweise US 6.277.951), aber auch eine aufwendige mehrstufige Schmelzekristallisation in Kombination mit einer Kristallisation aus Lösung für stärker verunreinigtes Dilactid wird in der GB 2.407.572 als Reinigungsprozess für das Rohlactid beschrieben. Aus dem Rohlactid aufwendig abgetrennt werden müssen vor allem meso-Dilactid sowie L-Milchsäure und die linearen Oligomere der Milchsäure. Milchsäure sowie ihre linearen Oligomere beeinflussen als OH- und COOH-gruppenhaltige Verbindungen vor allem die Ringöffnungspolymerisation des L,L-Dilactids durch Regelung der Molmasse infolge Kettenabbruchs. Meso-Dilactid bringt D-Lactideinheiten in das Makromolekül ein, die als "Störstellen" dann zu morphologischen Änderungen im Festkörper mit den bekannten Verringerungen in der Kristallinität und den damit zusammenhängenden Erniedrigungen des Schmelzpunktes oder der Festigkeit führen.

Zur zyklisierenden Depolymerisation wird eine niedermolekulare Oligo-L-milchsäure eingesetzt, die in der Mehrzahl der bekannt gemachten Verfahren durch Polyveresterung von L-Milchsäure erhalten wird. In der CA 2.128.509 wird eine Verfahrensvariante beschrieben, bei der L-Milchsäurealkylester durch Polyumesterung oligomerisiert werden.

Die Polykondensation der L-Milchsäure ist eine typische Polykondensationsreaktion eines Monomers vom AB-Typ (OH- und COOH-Gruppe an einem Monomer) mit Abspaltung von H₂O als niedermolekulare Komponente bei Aktivierung durch einen A_{Ac}2-Mechanismus. Entsprechend den für A_{Ac}2-Reaktionen typischen Reaktionsschritten erfolgt primär Addition elektrophiler Katalysatoren, vorzugsweise von Protonen, an die Carbonylgruppe (COOH-Gruppe der Milchsäure, Lactoylesterendgruppen bei oligomeren bzw. polymeren Milchsäuren) mit nachfolgender Addition nucleophiler Reaktionspartner (OH-Gruppen der Milchsäure, OH-Gruppen von Lactoylesterendgruppen im Falle der linearen Oligomere oder Polymere) an den Carbonylkohlenstoff und Eliminierung des ursprünglichen Substituenten. Prinzipiell sollten auch Kationen bzw. Komplexe ausgewählter Metalle, wie sie für Polykondensationen vom AA/BB-Typ (Umsetzung von Diolen mit Dicarbonsäuren) eingesetzt werden, diese Polyveresterung der Milchsäure aktivieren. Insbesondere für die Herstellung der Polyalkylenterephthalate (PET, PBT, PTT) werden Mangan, Calcium, Antimon, Titan oder Zinn in Form der Oxide, Alkoxide oder Acetate eingesetzt, wobei die Auswahl aus diesem Pool von metallbasierten Katalysatoren engen, vom Polyester und der Technologie definierten Grenzen unterliegt. So wird beispielsweise Polyethylenterephthalat (PET) seit langem ausschließlich in Gegenwart von Sb-Verbindungen (Sb₂O₃ oder Sb(ac)₃) hergestellt, während für Polybutylenterephthalat (PBT) überwiegend Titanalkoxide als Katalysatoren genutzt werden. Die katalytisch hochaktiven Titanalkoxide sind für PET infolge fehlender Hydrolysestabilität im Direktveresterungsprozess der Terephthalsäure mit Ethylenglykol und Gelbverfärbung des Polymers nicht geeignet. Erst neuerdings kann PET auch in Gegenwart spezieller Titanverbindungen synthetisiert werden (DE 10337522). In dieser Patentschrift werden Komplexe des Titan, Zirkonium und Hafnium als Katalysatoren für die Herstellung von aromatisch-aliphatischen Polyestern des AA/BB-Typs beansprucht. Mit Polykondensationstemperaturen von 250 °C (PBT) und 280 °C (PET) und in einem weitgehend wasserfreien Reaktionssystem, bestehend aus Terephthalsäure und einem Alkandiol (Ethylenglykol, Butandiol), erfolgt der Einsatz dieser Katalysatoren allerdings unter signifikant anderen Bedingungen im Vergleich zur Polykondensation der Milchsäure. Insbesondere der Einsatz einer 80-85%igen wässrigen Milchsäure und die um ca. 100 °C erniedrigten Prozesstemperaturen sind dabei zu nennen.

Die in der präparativen organischen Chemie üblicherweise eingesetzten anorganischen (H₂SO₄) oder organischen Säuren (RSO₃H) sind infolge paralleler Aktivierung der Etherbildung als Katalysatoren der Milchsäurepolykondensation ungeeignet, da infolge der thermodynamischen Favorisierung von sechsgliedrigen Ringsystemen bei der zyklisierenden Depolymerisation auch diese Ether zyklisieren können. Diese zyklischen Ether wären dann zusätzliche Verunreinigungen des Dilactids mit schwieriger Abtrennbarkeit und Potenzial zur Störung der Ringöffnungspolymerisation sowie negativer Beeinflussung der molekularen Parameter des Endprodukts.

Für die zyklisierende Depolymerisation der Oligo- bzw. Polymilchsäure zu Dilactid gelten vorrangig die thermodynamischen Grundlagen der Ring/Ketten-Gleichgewichte zyklischer Ester (Lactone) und Amide (Lactame), wie sie beispielsweise in H.-G. Elias: Makromoleküle, 6. Aufl., Weinheim: Wiley-VCH, 2002, ausführlich behandelt werden. Im Gegensatz zur autokatalysierten Polykondensation der Milchsäure zu einem niedermolekularen Polyester werden zur Beschleunigung der zyklisierende Depolymerisation dieser Oligo- bzw. Polymilchsäure zu Dilactid Katalysatoren, vorzugsweise Zinn(II)-salze und Zinn(II)-oxid, in allen beschriebenen Verfahren eingesetzt. Die Aktivierung der zyklisierenden Depolymerisation erfolgt analog der Polykondensation durch Addition elektrophiler Katalysatoren mit nachfolgender Addition der nucleophilen OH-Endgruppe des Polyesters an den Estercarbonylkohlenstoff und Eliminierung unter Ringbildung. Dabei können intermediär durch diesen Backbiting-Prozess (H. Kricheldorf et al., Macromol. Chem., Macromol. Symp. 32 (1990) 285) durchaus auch Makrozyklen gebildet werden, die dann zum dimeren Zyklus weiter gespalten werden.

### Aufgabe der Erfindung

Aufgabe der Erfindung ist die Bereitstellung eines verbesserten, wirtschaftlich effizienten Verfahrens zur Herstellung von L,L-, D,D-, D,L- bzw. meso-Dilactid, umfassend die Polykondensation von L-, D- oder D,L-Milchsäure zu einem höhermolekularen Polyester der L-, D- bzw. D,L-Milchsäure (Polymilchsäuren) und die zyklisierende Depolymerisation der Polymilchsäuren zu den Dilactiden.

### Beschreibung der Erfindung

Erfindungsgemäß wird dies dadurch erreicht, dass die höhermolekularen Polyester der L-, D- bzw. D,L-Milchsäure für die zyklisierende Depolymerisation in Gegenwart hydrolysestabiler Metallverbindungen als Katalysator hergestellt werden, wobei als hydrolysestabile Katalysatoren für die Polykondensation der Milchsäuren bzw. Polyumesterung der Milchsäureester Komplexe von Titan bzw. Zirkonium entsprechend der Struktur 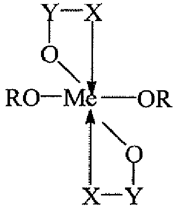 verwendet werden, wobei
Me = Ti, Zr;
R = -H, -Alkyl, -Aryl, -PO(OR')₂, -HPOOR', -SO₂R';
X = >O, >S; und
Y = >CH-, >C<, 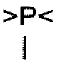
sind
und wobei die chelatbildenden Komponenten
- Acetylacetonate
- Alkali- und Ammoniumlactate
- Phosphorsäure- und Pyrophosphorsäureester
- Phosphorigsäureester
- Ethanolamine und Ethanoldiamine
sind,
und dass die Polymilchsäuren bei der Polykondensation auf Molmassen zwischen 6.000 g/mol und 10.000 g/mol, vorzugsweise zwischen 7.000 g/mol und 10.000 g/mol, insbesondere zwischen 8.000 g/mol und 10.000 g/mol, polymerisiert werden.

Die Katalysatoren sind durch hohe Stabilität charakterisiert, so dass bei chargenweiser Versuchsdurchführung mit im Anschluss an die Polykondensation erfolgender Depolymerisation im gleichen Reaktor eine mehrmalige Dosierung von Milchsäure möglich ist, ohne dass der Katalysator nachdosiert werden muss. Bei kontinuierlicher Verfahrensweise und Überführung des Polykondensats in einen separaten Depolymerisationsreaktor muss naturgemäß der Katalysator ergänzt werden.

In der US 6.277.951 wird ausgeführt, dass bei höheren Molmassen die Razemisierung in Form der Bildung von meso-Dilactid zunimmt. So beträgt der meso-Dilactidgehalt bei Einsatz eines Ausgangsoligomers mit Mₙ = 520 g/mol ca. 5,3 %. Wird die Molmasse des Präpolymers auf 2.500 g/mol erhöht, so steigt parallel dazu der meso-Dilactidgehalt im Destillat auf ca. 11 %. Darüber hinaus sollen Kationen insgesamt zu verstärkter meso-Dilactidbildung führen.

Ergebnisse der Untersuchungen zur zyklisierenden Depolymerisation der nach dem erfindungsgemäßen Verfahren hergestellten Polymilchsäuren sind in den Tabellen 1 bis 3 zusammengefasst. Die ermittelten effektiven Geschwindigkeitskonstanten der Depolymerisation sind komplexer Natur, und sie resultieren aus der Überlagerung der im System ablaufenden chemischen Reaktionen mit der Stoffübertragung des Dilactids aus der Schmelze in die Gasphase. Als chemische Reaktionen, die die Geschwindigkeit der Dilactidbildung determinieren, sind neben der eigentlichen Depolymerisation vor allem die Ringöffnungspolymerisation, entsprechend dem Gleichgewichtsgewichtscharakter der PLA-Bildung, sowie die Hydrolyse des Dilactids als wichtigste Nebenreaktion zu nennen. Dieser Systemabhängigkeit Rechnung tragend sind in Tabelle 1 die relativen Geschwindigkeiten für die gewählte Versuchsmethodik aufgeführt.

**Tabelle 1. Effektive Geschwindigkeitskonstanten der Dilactidbildung in Abhängigkeit von der Molmasse des Präpolymers**

| **Mₙ [g/mol]** | **Katalysator** | **Rel. Prozessgeschwindigkeit** |
|---|---|---|
| 550 | - | 1,0 |
| 800 | + | 1,1 |
| 3500 | + | 3,8 |
| 6100 | + | 9,2 |
| 7500 | + | 12,0 |
| 10000 | + | 14,1 |

COOH-Gruppenkonzentration und Drehwinkel sind Ausdruck der Reinheit des Rohlactids, wobei die COOH-Gruppenkonzentration direkt lineare Oligomere, vorzugsweise Lactoylmilchsäure, anzeigt. Sie können sowohl durch estertypische Umesterungsreaktionen unter Beteiligung der Endgruppen als auch durch Hydrolyse des Dilactids gebildet werden. Die optische Aktivität, gemessen am Drehwinkel, zeigt sowohl chemische als auch optische Verunreinigungen des Dilactids an.

**Tabelle 2. COOH-Konzentration im Roh-L,L-dilactid in Abhängigkeit von der Molmasse des Präpolymers (Depolymerisationstemperatur: 200 °C)**

| **Mₙ [g/mol]** | **[COOH]** [**mmol/g**] |
|---|---|
| 550 | 1,20 |
| 800 | 0,75 |
| 3500 | 0,21 |
| 6100 | 0,13 |
| 7500 | 0,08 |
| 10000 | 0,06 |

**Tabelle 3. Drehwinkel des Roh-L,L-dilactids in Abhängigkeit von der Molmasse des Präpolymers (Depolymerisationstemperatur: 200 °C)**

| **Mₙ [g/mol]** | **[α] [grd]** |
|---|---|
| 550 | -235 |
| 800 | -247 |
| 3500 | -255 |
| 6100 | -257 |
| 7500 | -260 |
| 10000 | -260 |

Im Vergleich dazu beträgt der Drehwert von reinem L,L-Dilactid -282°. Es ist deutlich zu erkennen, dass ein höheres Molekulargewicht zu einem reineren Produkt führt.

In weiterer Ausbildung der Erfindung können die Polymilchsäuren durch Polykondensation von Milchsäure oder Polyumesterung von Milchsäureestern hergestellt werden. Die im erfindungsgemäßen Verfahren eingesetzten Katalysatoren können auch zur Aktivierung der Polyumesterung von Milchsäureestern zu Polymilchsäuren entsprechend Gl. (2) eingesetzt werden.

n CH₃CH(OH)COOR ↔ [-OCH(CH₃)CO-]ₙ + (n-1) ROH Gl. (2)

mit R:
-C₂H₅; -C₃H₇-; -C₂H₄OH.

Auch die Polykondensation von Milchsäure/Milchsäureester-Gemischen wird durch diese Katalysatoren beschleunigt. Milchsäureester bilden eine Alternative zu den klassischen Verfahren der Milchsäurereinigung durch Membran- oder Fällungsprozesse. Sie können destillativ aus der Rohmilchsäure nach Veresterung mit den Alkanolen abgetrennt werden.

Im Verfahren der vorliegenden Erfindung werden die Polymilchsäuren in Gegenwart des hydrolysestabilen Katalysators auf Molmassen zwischen 800 g/mol und 10.000 g/mol, vorzugsweise 6.000 g/mol und 10.000 g/mol, noch bevorzugter zwischen 7.000 g/mol und 10.000 g/mol, insbesondere zwischen 8.000 g/mol und 10.000 g/mol, polymerisiert. Durch diese Erhöhung des bei der Polykondensation erzielbaren Molekulargewichts kann eine Depolymerisation zu reineren Produkten durchgeführt werden, so dass nachfolgende Reinigungsschritte weniger aufwendig werden oder mitunter sogar gänzlich entfallen können.

Die hydrolysestabilen Metallverbindungen werden aus Metallverbindungen von Titan und Zirkonium ausgewählt. Durch den Einsatz dieser Verbindungen ist es erstmals möglich, höhermolekulare Polymilchsäuren mit zahlenmittleren Molmassen bis 10.000 g/mol und hoher Prozessgeschwindigkeit durch Polykondensation bei schonenden Temperaturen herzustellen und diese dann der Depolymerisationsstufe zuzuführen. Im Gegensatz zu der dem Stand der Technik entsprechenden autokatalysierten Polykondensation der L-, D- oder D,L-Milchsäure bleibt die Katalysatorkonzentration bei der fremdkatalysierten Polykondensation über den gesamten Prozess unverändert. Die COOH-Gruppen- und damit die Protonenkonzentration verringert sich bei der Molmassenzunahme infolge abnehmender Endgruppenkonzentration rasch. Parallel dazu nimmt bei diesen durch COOH-Gruppen autokatalysierten Reaktionen die Geschwindigkeit mit zunehmendem Umsatz der COOH-Gruppen rasch ab.

Der Einsatz von Zinn(II)-halogeniden bzw. Zinn(II)-carboxylaten oder Zinn(II)-oxid bei der Oligomerisierung von Milchsäure, die entsprechend dem Stand der Technik als Katalysatoren der Ringöffnungspolymerisation sowie der zyklisierenden Depolymerisation bei nahezu allen bekannt gemachten Verfahren eingesetzt werden (vgl. die bereits aufgeführten US-, GB- und CA-Patentschriften), würde zwar die Unabhängigkeit der fremdkatalysierten Polykondensation von der Endgruppenkonzentration nutzen, aber als typische Polymerisations- bzw. Depolymerisationskatalysatoren beschleunigen sie bereits bei niedrigen mittleren Molmassen vorzugsweise die Dilactidund nicht die Polymerbildung. Dementsprechend erfolgt die Dilactidbildung in der GB 2.407.572 ab Molmassen von 400 g/mol (Bereich 400 bis 2.000 g/mol) (Prozess autokatalysiert durch COOH der L-Milchsäure: L-Lactid im Vorkondensat 97,6 %, fremdkatalysiert: L-Anteil im Dilactid 87,8 %). Folge der Dilactidbildung unter Einsatz eines sehr niedermolekularen Präpolymers ist ein hoher Anteil von die spätere Polymerisation störenden Verunreinigungen im Rohlactid, die durch ein aufwendiges mehrmaliges Kristallisationsverfahren abgetrennt werden müssen. Verursacht werden diese Verunreinigungen durch Wasser, das infolge der geringen Molmassen durch parallel ablaufende Polykondensationsprozesse noch in hohen Anteilen bei der Depolymerisation gebildet wird und bereits gebildetes Dilactid in der Schmelze bzw. in den Destillaten zu Lactoylmilchsäure oder Milchsäure hydrolysieren kann. Entsprechend Gl. (3) werden aus n Mol Milchsäure bei der Polykondensation (n-1) Mol Wasser gebildet. Unter Berücksichtigung des Zusammenhangs von zahlenmittlerem Polymerisationsgrad und Reaktionsfortschrittsgrad in den Gl. (4a) und (4b) bedeutet dies beispielsweise, dass aus 900 g (10 mol) einer wasserfreien Milchsäure insgesamt 162 g Wasser gebildet werden, entsprechend 81 g bei Dimerisation oder ca. 146 g, wenn das lineare Decamer mit Mₙ = 738 g/mol hergestellt wird. Für die gleiche Milchsäuremenge bei Herstellung eines linearen Oligomers mit Mₙ = 3.000 g/mol (entsprechend Pₙ = 41,6) müssen ca. 158 g Wasser aus dem System entfernt werden. 12 g Wasser stehen damit selbst beim Übergang von Pₙ = 10 auf Pₙ = 41,6 noch für die Dilactidhydrolyse zur Verfügung. Das entspricht einem Hydrolysepotenzial von 0,67 mol/mol bei Abtrennung und Aufarbeitung des Dilactids.

n CH₃CH(OH)COOH ↔ [-OCH(CH₃)CO-]ₙ + (n-1) H₂O Gl. 3

Pₙ = 1/(1-p) Gl. 4a

p = 1 - 1/Pₙ Gl. 4b

Die hohe Aktivität der erfindungsgemäßen Katalysatoren erlaubt einen sehr geringen mengenmäßigen Einsatz von 5·10⁻⁵ bis 1·10⁻²mol Katalysatormetall/mol Monomereinheit. Über Katalysatorstruktur und Katalysatorkonzentration lassen sich sowohl die Polykondensationsgeschwindigkeit als auch die in der Schmelze maximal erreichbare Molmasse steuern. Für vergleichbare Prozessbedingungen besteht ein nahezu linearer Zusammenhang zwischen erreichbarer Molmasse und Konzentration des Katalysators (Tabelle 4).

**Tabelle 4. Mₙ-Werte von L-Milchsäurepolykondensaten in Abhängigkeit von der Katalysatorkonzentration**

| Polykondensationsbedingungen: T: 180 °C; p: 1,6 kPa; t: 4 h | |
|---|---|
| **Kat.-Konz.** * **10⁴ [mol/mol]** | **Mₙ [g/mol]** |
| ohne | 550 |
| 0,1 | 800 |
| 0,75 | 1600 |
| 1,0 | 2300 |
| 3,5 | 3400 |
| 6,0 | 4800 |
| 8,5 | 7500 |
| 10,5 | 10000 |

Im Gegensatz zum Stand der Technik wurde gefunden, dass die im erfindungsgemäßein Verfahren eingesetzten Ti- und Zr-Katalysatoren nicht nur die Polykondensation beschleunigen und zu höheren Molmassen bei der durch Polykondensation hergestellten Poly-L-milchsäure führen, sondern diese Poly-L-milchsäuren höherer Molmasse auch schneller depolymerisieren und zu einem reineren Rohlactid führen. Die höhere Molmasse führt zu reduzierten Endgruppenkonzentrationen und damit auch zur Reduktion von Parallel- und Folgereaktionen der Depolymerisation. Als wichtigste Parallelreaktion der zyklisierenden Depolymerisation ist unter den gewählten Bedingungen (hohe Temperatur, Vakuum) die Polykondensation der OH- und COOH-Endgruppen anzusehen, in deren Verlauf Wasser gebildet wird. Dieses wiederum kann dann in einem Folgeschritt bereits gebildetes Dilactid in der Reaktionsschmelze sowie in den Brüden hydrolysieren (Schema 2). Beide Reaktionen verringern die Prozessgeschwindigkeit der Dilactidbildung.

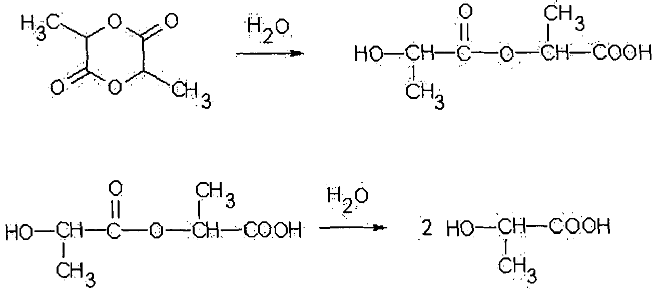

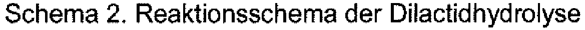

Im erfindungsgemäßen Verfahren werden als hydrolysestabile Katalysatoren für die Polykondensation der Milchsäuren bzw. Polyumesterung der Milchsäureester Komplexe des Titan bzw. Zirkonium entsprechend der nachstehenden Struktur verwendet: 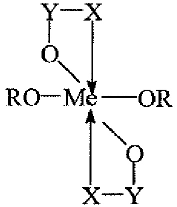 worin:
Me = Ti, Zr;
R = -H, -Alkyl, -Aryl, -PO(OR')₂, -HPOOR', -SO₂R';
X = >O, >S; und
Y = >CH-, >C<, 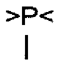
sind.

Es wird somit vorgeschlagen, dass die Herstellung der niedermolekularen Poly-L-milchsäure in Gegenwart eines Veresterungs- bzw. Umesterungskatalysators erfolgt, der ausgewählt ist aus Metallchelaten der IV. Nebengruppe des Periodensystems der Elemente mit der in Schema 3 dargestellten allgemeinen Struktur.

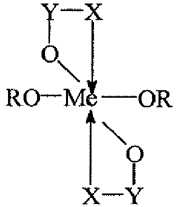

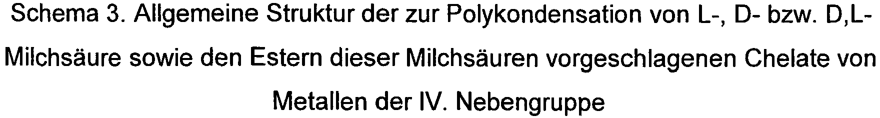

Darin haben Me, R, X und Y die obige Bedeutung.

Als chelatbildende Komponenten für die in Schema 3 aufgeführten Strukturen sind vorzugsweise einzusetzen:
- Acetylacetonate
- Alkali- und Ammoniumlactate
- Phosphorsäure- und Pyrophosphorsäureester
- Phosphorigsäureester
- Ethanolamine und Ethanoldiamine.

Mit Dihydroxybis(ammoniumlactato)titanat (Tyzor LA, DuPont) (Schema 4, Mitte), Isopropyltri(dioctylphosphato)titanat (KR 12, Kenrich Petrochemicals), Isopropyldioctylphosphatodi(octylpyrophosphato)titanat (KR 38 S, Kenrich Petrochemicals) (Schema 4, rechts) und Diisopropylbis(acetylacetonato)titanat (Schema 4, links) steht eine ganze Reihe dieser Chelatkomplexe von Titan zur Verfügung, wobei die Auswahl die katalytisch aktiven Verbindungen nicht begrenzt, sondern lediglich der Veranschaulichung der zur Verfügung stehenden Strukturvielfalt dient.

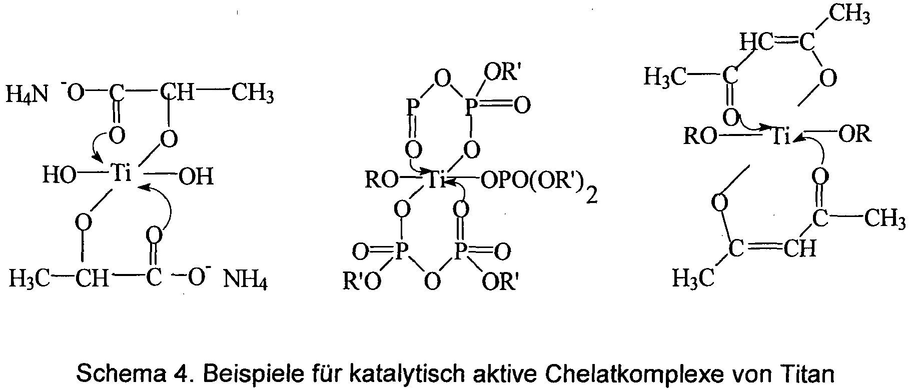

Chelatstrukturen des in Schema 3 dargestellten Typs sind koordinativ abgesättigt, so dass sie im Gegensatz zu Titan- und Zirkoniumalkoxiden ausreichende hydrolytische und thermische Stabilität aufweisen und damit auch unter den technischen Prozessbedingungen der Polykondensation der L-, D- oder D,L-Milchsäure bzw. ihrer Ester nicht geschädigt oder deaktiviert werden.

Die zur Beschleunigung der Polykondensation von Milchsäuren im erfindungsgemäßen Verfahren eingesetzten Titan- und Zirkoniumverbindungen sind als Handelsprodukte der Fa. DuPont bzw. Kenrich Petrochemicals kommerziell verfügbar.

In einer bevorzugten Ausführungsform der Erfindung können die zur Katalyse eingesetzten Komplexe des Titan oder Zirkonium somit durch die jeweils ausgewählten Liganden weitere Funktionalitäten aufweisen, die zur weiteren Verbesserung der Katalyse und zu höheren Ausbeuten beitragen können.

In einer anderen bevorzugten Ausführungsform der Erfindung können zur Herstellung höhermolekularer Polyester der L-, D- bzw. D,L-Milchsäure die obigen Katalysatoren auch in Kombination mit technologie- und/oder strukturadäquaten Cokatalysatoren eingesetzt werden, solange die Wirkung der Erfindung nicht beeinträchtigt wird. Die erfindungsgemäßen Katalysatoren können beispielsweise in Kombination mit bekannten Ver- und Umesterungskatalysatoren ohne Einbuße ihrer katalytischen Effektivität eingesetzt werden. Geeignete, technologie- und applikationsadäquate Systemkomponenten sind etwa Zinn(II)-halogenide, Zinn(II)-carboxylate und Zinn(IV)-alkoxide sowie Verbindungen von Zink und Antimon. In Abhängigkeit von der hydrolytischen Stabilität dieser Cokatalysatoren können mit diesen Katalysatorkombinationen auch Milchsäuren mit höheren Wassergehalten direkt polykondensiert werden, wobei als zusätzlicher Effekt eine Reduktion der Flüchtigkeit von Milchsäure bei Entwässerung und Polykondensation beobachtet wird.

In einer Ausführungsform der Erfindung können die Rohdilactide nach üblichen Verfahren durch Rektifikation, Schmelze- oder Lösungskristallisation weiter gereinigt werden. Dadurch können bereits bekannte und in den Laboratorien bzw. Fabriken bereits vorhandene Vorrichtungen auch für das erfindungsgemäße Verfahren verwendet werden. Neuinvestitionen entfallen somit.

Die Depolymerisation der durch Polykondensation hergestellten Polymilchsäuren erfolgte dem Stand der Technik gemäß in Gegenwart von Zinn(II)-chlorid oder Zinn(II)-octonoat bei Temperaturen von 180 bis 240 °C unter einem Vakuum von 133 Pa mit Abtrennung des gebildeten Dilactids über eine beheizte Kolonne. Ebenfalls dem bekannten Stand der Technik entsprechend kann das Rohlactid destillativ oder durch Kristallisation gereinigt werden.

Beschrieben hierin ist ein nach dem erfindungsgemäßen Verfahren hergestelltes L,L-, D,D-, D,L- bzw. meso-Dilactid mit den oben angeführten vorteilhaften Eigenschaften, insbesondere der hohen Stereoselektivität.

Beschrieben hierin ist die Verwendung eines solchen L,L-, D,D-, D,L- bzw. meso-Dilactids zur Herstellung von Polyestern der L-, D- bzw. D,L-Milchsäure, vorzugsweise von Polyestern mit zahlenmittleren Molmassen Mₙ größer 125.000 g/mol und molekularen Uneinheitlichkeiten M_{w}/Mₙ zwischen 1,6 und 3,0. Die Herstellung solcher Polyester der Milchsäure mit hohem Molekulargewicht und einheitlicher Molmassenverteilung ist aufgrund der hohen Stereoselektivität der nach dem erfindungsgemäßen Verfahren erhaltenen Dilactide problemlos möglich.

Mit den erfindungsgemäß hergestellten Dilactiden wurden Polymerisationsversuche mit diskontinuierlicher und kontinuierlicher Verfahrensweise durchgeführt, um ihre Eignung für die Polymerherstellung zu prüfen. Für die diskontinuierlichen Polymerisationsversuche wurde eine Glasapparatur mit geschraubtem Blattrührer eingesetzt. Für die kontinuierlichen Polymerisationsversuche wurde ein Doppelschneckenextruder mit einem speziell auf diese Massepolymerisation abgestimmten Schneckendesign verwendet. In Gegenwart von Zinn(II)-octonoat konnten dabei in Abhängigkeit von den Polymerisationsbedingungen (Temperatur, Cokatalysatorzusatz, Stabilisierung) und der Verfahrensweise (diskontinuierlich im Rührreaktor oder Horizontalkneter, kontinuierlich im Doppelschneckenextruder) Polymere mit zahlenmittleren Molmassen im Bereich 40.000 g/mol < Mₙ < 175.000 g/mol bei molekularen Uneinheitlichkeiten von 1,5 < M_{w}/Mₙ < 3,0 synthetisiert werden.

Die Erfindung wird anhand der folgenden Beispiele beschrieben, die lediglich zur Veranschaulichung und nicht als Einschränkung dienen sollen.

### Beispiele

### Beispiel 1 (Polykondensation, Vergleichsbeispiel)

1060 g einer 85%igen L-Milchsäure (Versuchsprodukt der Anmelderin) werden in einer Glasapparatur mit Rührer, Außenheizung und temperierbarer Vigreuxkolonne im Vakuum bei 155 °C innerhalb von 2 h vollständig entwässert, wobei das Vakuum so geregelt wird, dass keine Milchsäure über das Destillat abgeführt wird. Nach der Entwässerungsphase wird die Temperatur auf 185 °C erhöht und bei 13 kPa 6 h lang polykondensiert. Von dem Polykondensationsprodukt werden Ausbeute, Molmasse sowie [COOH]-Gehalt bestimmt.

| | |
|---|---|
| Ausbeute: | 725 g |
| Mₙ: | 550 g/mol |
| [COOH]: | 1,9 mmol/g |

### Beispiel 2 (Polykondensation, erfindungsgemäß)

1060 g einer 85%igen L-Milchsäure (Versuchsprodukt der Anmelderin) werden analog Beispiel 1 in einer Glasapparatur mit Rührer, Außenheizung und temperierbarer Vigreuxkolonne im Vakuum bei 155 °C innerhalb von 2 h vollständig entwässert, wobei das Vakuum so geregelt wird, dass keine Milchsäure über das Destillat abgeführt wird. Nach der Entwässerungsphase wird die Temperatur auf 185 °C erhöht und bei 13 kPa unter Zusatz von 10⁻⁴ mol/mol Dihydroxybis(ammoniumlactato)titan (Tyzor^{®} LA, DuPont) (0,6 g einer 50%igen Lösung) 4 h lang polykondensiert. Von dem Polykondensationsprodukt werden analog Beispiel 1 Ausbeute, Molmasse sowie der [COOH]-Gehalt bestimmt.

| | |
|---|---|
| Ausbeute: | 730 g |
| Mₙ: | 2.300 g/mol |
| [COOH]: | 0,4 mmol/g |

### Beispiel 3 (Polykondensation, erfindungsgemäß)

Analog Beispiel 2 werden 1060 g einer 85%igen L-Milchsäure (Versuchsprodukt der Anmelderin) in einer Glasapparatur mit Rührer, Außenheizung und temperierbarer Vigreuxkolonne im Vakuum bei 155 °C innerhalb von 2 h vollständig entwässert, wobei das Vakuum so geregelt wird, dass keine Milchsäure über das Destillat abgeführt wird. Nach der Entwässerungsphase wird die Temperatur auf 200 °C erhöht und bei 13 kPa unter Zusatz von 3*10⁻⁴ mol/mol Isopropyltri(dioctylphosphato)titanat (KR^{®}12, KENRICH Petrochemicals) (3,2 g) 3 h lang polykondensiert.

| | |
|---|---|
| Ausbeute: | 730 g |
| Mₙ: | 3.600 g/mol |
| [COOH]: | 0,3 mmol/g |

### Beispiel 4 (Polykondensation, erfindungsgemäß)

In einem flüssigbeheizten 25-1-Laborrührreaktor, ausgerüstet mit Ankerrührer, Mantelheizung, Bodenventil und temperierbarer Kolonne sowie mit Erfassung von Innenund Manteltemperatur, werden 15 kg einer 65%igen L-Milchsäure im Vakuum bei 120 bis 160 °C (brüdengesteuertes Temperaturprogramm) entwässert und anschließend in Gegenwart von 3,0*10⁻⁴ mol/mol Dihydroxybis(ammoniumlactato)titan (22g einer 50%igen Lösung) 4 h lang bei 190 °C polykondensiert.

| | |
|---|---|
| Ausbeute: | 10 kg |
| Mₙ: | 3.400 g/mol |
| [COOH]: | 0,4 mmol/g |

### Beispiel 5 (Polykondensation, erfindungsgemäß)

Analog Beispiel 4 werden in einem flüssigbeheizten 25-1-Laborrührreaktor, ausgerüstet mit einem Ankerrührer, Mantelheizung, Bodenventil und temperierbarer Kolonne sowie mit Erfassung von Innen- und Manteltemperatur, 15 kg einer 65%igen L-Milchsäure im Vakuum bei 120 bis 160 °C (brüdengesteuertes Temperaturprogramm) entwässert und anschließend in Gegenwart einer Katalysatorkombination, bestehend aus 2,0*10⁻⁴ mol/mol Dihydroxybis(ammoniumlactato)titan und 2*10⁻⁴ mol/mol SnCl₂, 4 h lang bei 190 °C polykondensiert.

| | |
|---|---|
| Ausbeute: | 10 kg |
| Mₙ: | 4.800 g/mol |
| [COOH]: | 0,2 mmol/g |

### Beispiel 6 (Polykondensation, erfindungsgemäß)

Analog Beispiel 2 werden 1060 g einer 85%igen L-Milchsäure (Versuchsprodukt der Anmelderin) entwässert und unter Zusatz einer Katalysatorkombination, bestehend aus 2*10⁻⁴ mol/mol Isopropyltri(dioctylpyrophosphato)titanat (KR^{®}38, KENRICH Petrochemicals) (2,6 g) und 3*10⁻⁴ mol/mol Sn(C₂O₄), 3 h lang polykondensiert. Von dem Polykondensationsprodukt werden analog Beispiel 1 Ausbeute, Molmasse sowie der [COOH]-Gehalt bestimmt.

| | |
|---|---|
| Ausbeute: | 730 g |
| Mₙ: | 6.100g/mol |
| [COOH]: | 0,13 mmol/g |

### Beispiel 7 (Polykondensation, erfindungsgemäß)

900 g einer wasserfreien L-Milchsäure (Versuchsprodukt der Anmelderin) werden in einer Glasapparatur mit Rührer, Außenheizung und temperierbarer Vigreuxkolonne im Vakuum bei Temperaturen von 150-210 °C in Gegenwart von 5,5*10⁻⁴ mol/mol Isopropyltri(dioctylphosphato)titanat (KR^{®}12, KENRICH Petrochemicals) (3,2 g) und 5*10⁻⁴ mol/mol SnCl₂ 5 h lang polykondensiert. Das Temperatur- und Vakuumprogramm ist so eingestellt, dass keine Milchsäure über das Destillat abgeführt wird. Das Produkt wird im Vakuum bei 155 °C innerhalb von 2 h vollständig entwässert.

Als Endvakuum werden 13 kPa gewählt. Von dem Polykondensationsprodukt werden analog Beispiel 1 Ausbeute, Molmasse sowie der [COOH]-Gehalt bestimmt.

| | |
|---|---|
| Ausbeute: | 730 g |
| Mₙ: | 10.000 g/mol |
| [COOH]: | 0,06 mmol/g |

### Beispiel 8 (Polykondensation, erfindungsgemäß)

Analog Beispiel 2 werden 920 g einer kommerziellen D,L-Milchsäure unter Zusatz von 2*10⁻⁴ mol/mol Dihydroxybis(ammoniumlactato)titan polykondensiert und aufgearbeitet. Von dem Polykondensationsprodukt werden analog Beispiel 1 Ausbeute, Molmasse sowie der [COOH]-Gehalt bestimmt.

| | |
|---|---|
| Ausbeute: | 700 g |
| Mₙ: | 2.600 g/mol |
| [COOH]: | 0,3 mmol/g |

### Beispiel 9 (Polyumesterung, erfindungsgemäß)

1180 g Ethyl-L-lactat (Versuchsprodukt der Anmelderin) werden in einer Glasapparatur mit Rührer, Außenheizung und temperierbarer Vigreuxkolonne bei 200 °C in Gegenwart von 3*10⁻⁴ mol/mol Dihydroxybis(ammoniumlactato)titan polykondensiert. Das abgespaltene Ethanol wird über die Kolonne abgeführt und kann für erneute Veresterungen von L-Milchsäure direkt verwendet werden. Zur vollständigen Abspaltung der Ethylestergruppen wird die Reaktion nach Abführung der Hauptmenge des sich bildenden Ethanols unter Vakuum fortgeführt.

| | |
|---|---|
| Ausbeute: | 700 g |
| Mₙ: | 3.500 g/mol |
| [COOH]: | 0,1 mmol/g |

### Beispiel 10 (zyklisierende Depolymerisation)

1000 g der entsprechend Beispiel 2 bis 8 hergestellten Poly-L-milchsäure werden in einer Glasapparatur mit Rührer, Außenheizung und temperierbarer Füllkörperkolonne mit ebenfalls temperierbarem Kühler unter einem Vakuum von 1,3-2,0 kPa in Gegenwart eines Zinn(II)-Katalysators erhitzt. Wird eine der beschriebenen zinn(II)-haltigen Katalysatorkombinationen bei der Polykondensation eingesetzt, ist kein weiterer Katalysatorzusatz erforderlich. Wurden zur Präpolymerherstellung nur die bevorzugten Titan- oder Zirkoniumchelate allein als Katalysator verwendet, so werden noch 2*10⁻⁴ mol/mol SnCl₂, Sn⁰ oder Sn(C₂O₂) als Depolymerisationskatalysator der Oligomerschmelze zugesetzt. In Abhängigkeit von der gewählten Depolymerisationsgeschwindigkeit wird der Reaktionsansatz dann auf 185-220 °C erhitzt und das gebildete L,L-Dilactid über die Kolonne abgeführt, wobei die Kühlertemperatur zur Vermeidung von Kristallisation auf 100 °C gehalten wird.

In Abhängigkeit vom Substrat und den gewählten Depolymerisationsbedingungen werden Roh-L,L-dilactide mit den in den Tabellen 3 und 4 bereits dargestellten Carboxylgruppengehalten bzw. Drehwinkeln [α] erhalten.

Zur Feinreinigung werden die Rohlactide zur Abtrennung von Restmilchsäure, linearen Oligomeren und Restwasser im Vakuum destilliert oder aus Essigsäureethylester in Gegenwart von CaCO₃ als Säure-Scavenger umkristallisiert. Das so destillativ oder durch Kristallisation gereinigte L,L-Dilactid für die Massepolymerisation ist durch folgende Daten charakterisiert:

| | |
|---|---|
| Tₘ: | 96 bis 97 °C |
| [α]: | -268 bis -270° |
| [COOH]: | 8 µmol/g |

Durch Destillation oder Kristallisation gereinigtes D,L-Dilactid weist folgende Daten auf:

| | |
|---|---|
| Tₘ: | 126 bis 127 °C |
| [COOH]: | 10 µmol/g |

### Beispiel 11 (Ringöffnungspolymerisation, diskontinuierlich, zylindrisches Glasgefäß)

36 g nach Beispiel 9 hergestelltes und gereinigtes sowie sorgfältig getrocknetes L,L-Dilactid werden in einem zylindrischen Glasreaktor mit einem randgängigen geschraubten Blattrührer (Schraubenrührer) unter Inertgas in einem Temperierbad aufgeschmolzen. Der aus Glas gefertigte und bis zum Boden reichende Schraubenrührer durchmischt die Schmelze axial. Der Rührer wird mit einer Drehzahl von 100 min⁻¹ betrieben. Nach Erreichen der Solltemperatur werden der gerührten Monomerschmelze 7,5*10⁻⁵ mol/mol Sn(oct)₂ in Form einer 0,1%igen Lösung in Toluol zugesetzt. Zur Ermittlung des Polymerisationsverlaufs können der Schmelze über einen seitlichen Ansatz Proben entnommen werden, von denen Momonerumsatz und Molmasse bestimmt werden. Die Umsatzbestimmung erfolgte gravimetrisch durch Umfällen der Polymilchsäuren aus Chloroform als Lösungsmittel und einem Methanol/Di-ethylether-Gemisch als Fällmittel. Die Molmassen wurden mittels GPC in CH₂Cl₂ ermittelt. Zur Kalibrierung wurden Polystyreneichstandards verwendet.

Nach einer Polymerisationszeit von 20 min wurde Folgendes ermittelt:
Monomerumsatz : U = 96 %
Molmasse: Mₙ = 60.000 g/mol
Molekulare Uneinheitlichkeit: Mₙ/M_{w} = 2,1

### Beispiel 12 (Ringöffnungspolymerisation im Doppelschneckenextruder)

Für die Massepolymerisation des L,L-Dilactids durch einen Reaktivextrusionsprozess wurde ein dichtkämmender gleichsinnig drehender Doppelschneckenextruder mit Innentemperatur- und Drehmomentregelung verwendet. Der eingesetzte Doppelschneckenextruder weist ein (L/D)-Verhältnis von 35 mit modular aufgebauter Schnecke auf, um flexibel die Schneckenkonfigurierung hinsichtlich Transport-, Knet- und Stauelementen den Prozessbedingungen und Endproduktparametern anpassen zu können.

1000 g in kristalliner Pulverform vorliegendes L,L-Dilactid, getrocknet im Vakuumtrockenschrank bei 40 °C über P₄O₁₀, wurde mit einer Lösung von Zinn(II)-octonoat in Toluol in einem Labortaumelmischer intensiv vermischt und anschließend das Toluol im Vakuum abgezogen. Die Katalysatorkonzentration betrug 3,0*10⁻⁴, 2,0*10⁻⁴ und 1,5*10⁻⁴mol/mol und entsprachen einem Zinngehalt von 247, 165 und 123 ppm.

Die Dosierung des Lactid/Katalysator-Gemischs erfolgte mittels volumetrischer Dosierung über Dosierschnecken.

| | |
|---|---|
| Umsatz: | 94% |
| Mₙ: | 112.000 g/mol |
| M_{w}: | 239.000 g/mol |

Aus der obigen Beschreibung geht klar hervor, dass unter Verwendung von erfindungsgemäßen hydrolysestablien Metallverbindungen als Katalysatoren Polymilchsäuren mit einem hohen Molekulargewicht und einem geringen COOH-Gehalt hergestellt werden konnten, die in weiterer Folge zu sehr reinen Dilactiden depolymerisiert werden konnten. Dadurch wurde ein effizientes Verfahren zur Herstellung von Dilactiden bereitgestellt, die aufgrund ihrer hohen Reinheit mit geringer bzw. gar keiner wieteren Reinigung zur Herstellung von Polyestern der Milchsäure verwendet werden können.

## Patentansprüche

1. Verfahren zur Herstellung von L,L-, D,D-, D,L- bzw. meso-Dilactid, umfassend die Polykondensation und/oder Polyumesterung von L-, D- oder D,L-Milchsäure bzw. Estern davon zu höhermolekularen Polyestern der L-, D- bzw. D,L-Milchsäure (Polymilchsäuren) und die zyklisierende Depolymerisation der Polymilchsäuren zu den Dilactiden,
**dadurch gekennzeichnet, dass** die höhermolekularen Polyester der L-, D- bzw. D,L-Milchsäure in Gegenwart hydrolysestabiler Metallverbindungen als Katalysator hergestellt werden,
wobei als hydrolysestabile Katalysatoren für die Polykondensation der Milchsäure bzw. Polyumesterung der Milchsäureester Komplexe von Titan bzw. Zirkonium entsprechend der Struktur 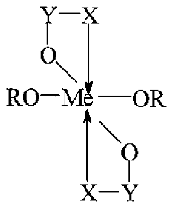 verwendet werden, wobei
Me = Ti, Zr;
R = -H, -Alkyl, -Aryl, -PO(OR')₂, -HPOOR', -SO₂R';
X = >O, >S; und
Y = >CH-, >C<, 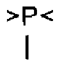
sind
und wobei die chelatbildenden Komponenten
- Acetylacetonate
- Alkali- und Ammoniumlactate
- Phosphorsäure- und Pyrophosphorsäureester
- Phosphorigsäureester
- Ethanolamine und Ethanoldiamine
sind,
und dass die Polymilchsäuren bei der Polykondensation auf Molmassen zwischen 6.000 g/mol und 10.000 g/mol, vorzugsweise zwischen 7.000 g/mol und 10.000 g/mol, insbesondere zwischen 8.000 g/mol und 10.000 g/mol, polymerisiert werden.

2. Verfahren nach Anspruch 1, **dadurch gekennzeichnet, dass** die Komplexe von Titan oder Zirkonium auf den ausgewählten Liganden weitere Funktionalitäten aufweisen.

3. Verfahren nach Anspruch 1 oder 2, **dadurch gekennzeichnet, dass** zur Herstellung höhermolekularer Polyester der L-, D- bzw. D,L-Milchsäure die Katalysatoren allein oder in Kombination mit technologie- und/oder strukturadäquaten Cokatalysatoren eingesetzt werden.

4. Verfahren nach einem der vorangegangenen Ansprüche, **dadurch gekennzeichnet, dass** die Rohdilactide nach üblichen Verfahren durch Rektifikation, Schmelze- oder Lösungskristallisation weiter gereinigt werden.

## Claims

1. A method for producing L,L-, D,D-, D,L- and meso-dilactides, comprising the polycondensation and/or polytransesterification of L-, D-, or D,L-lactic acid or of esters thereof to polyesters of higher molecular weights of L-, D- or D,L-lactic acid (polylactic acids) and cyclizing depolymerization of the polylactic acids to said dilactides,
**characterized in that** said polyesters of higher molecular weights of L-, D- and D,L-lactic acids are produced in the presence of hydrolysis-stable metal compounds as catalysts,
wherein, as hydrolysis-stable catalysts for the polycondensation of said lactic acids or for the polytransesterification of said lactic acid esters, complexes of titanium or zirconium of the following structure are used: 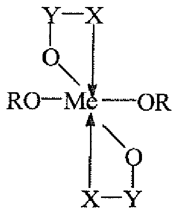 wherein
Me = Ti, Zr;
R = -H, alkyl, aryl, PO(OR')₂, -HPOOR', -SO₂R';
X = >O, >S; and
Y = >CH-, >C<, 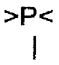
and wherein the chelating components are
- acetylacetonates
- alkali and ammonium lactates
- esters of phosphoric acid and pyrophosphoric acid
- esters of phosphorous acid
- ethanolamines and ethanoldiamines,
and **in that** said polylactic acids are polymerized by polycondensation to molecular masses between 6,000 g/mol and 10,000 g/mol, preferably between 7,000 g/mol and 10,000 g/mol, especially between 8,000 g/mol and 10,000 g/mol.

2. The process according to claim 1, **characterized in that** said complexes of titanium or zirconium have further functionalities on the selected ligands.

3. The process according to claim 1 or claim 2, **characterized in that** for producing polyesters of higher molecular weights of the L-, D-, or D,L-lactic acid, the catalysts are used alone or in combination with technologically and/or structurally appropriate co-catalysts.

4. The process according to any one of the preceding claims, **characterized in that** the raw dilactides are further purified by means of conventional methods by rectification, melt crystallization, or solution crystallization.

## Revendications

1. Procédé pour la production des L,L-, D,D- ou meso lactides, comprenant la polycondensation et/ou la polytransésterification des acides lactiques L, D ou D,L ou de leurs esters aux polyesters d'une masse moléculaire plus élevée des acides lactiques L, D ou D,L (acides polylactiques) et la dépolymérisation cyclisante des acides polylactiques aux lactides,
**caractérisé en ce que** les polyesters d'une masse moléculaire plus élevée des acides lactiques L, D ou D,L sont produits en présence des composés métalliques, résistants à l'hydrolyse, comme catalyseurs,
des complexes de titane ou de zirconium, ayant la structure suivante, étant utilisés comme les catalyseurs résistants à l'hydrolyse pour la polycondensation des acides lactiques et la polytransésterification des esters des acides lactiques 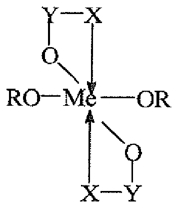 où
Me: Ti, Zr;
R: -H, -alkyle, -aryle, -PO(OR')₂, -HPOOR', -SO₂R';
X: -O-, -S-; et
Y: >CH-, >C<, >P<
et les composant chélateurs étant
- des acétylacétonates
- des lactates alcalins et des lactates d'ammonium
- des esters de l'acide phosphorique et de l'acide pyrophosphorique
- des esters de l'acide phosphoreux
- des éthanolamines et des éthanoldiamines
et **en ce que** les acides polylactiques sont polymérisés par la polycondensation à des masses moléculaires entre 6.000 g/mol et 10.000 g/mol, de préférence entre 7.000 g/mol et 10.000 g/mol, particulièrement entre 8,000 g/mol et 10,000 g/mol.

2. Procédé selon la revendication 1, **caractérisé en ce que** les complexes de titane ou de zirconium ont des fonctionnalités additionnelles sur les ligands sélectionnés.

3. Procédé selon la revendication 1 ou 2, **caractérisé en ce que**, pour la production des polyesters d'une masse moléculaire plus élevée des acides lactiques L, D ou D, L, les catalyseurs sont utilisés seuls ou en combinaison avec des co-catalyseurs technologiquement et/ou structurellement adéquats.

4. Procédé selon une des revendications précédentes, **caractérisé en ce que** les lactides bruts sont purifiés par des procédés habituels par la rectification, la cristallisation par fusion et la cristallisation par solution.
